# EUROPÄISCHE PATENTSCHRIFT

(11) **EP 0 446 204 B1**
(45) Veröffentlichungstag und Bekanntmachung des Hinweises auf die Patenterteilung: **10.02.1993**
(21) Anmeldenummer: 89907064.3
(22) Anmeldetag: 21.06.1989
(51) Int. Cl.: A61M 29/02

(54) **DILITATIONSKATHETER**
DILATATION CATHETER
CATHETER DE DILATATION

(30) Priorität: 25.06.1988 DE 3821544
(43) Veröffentlichungstag der Anmeldung: 18.09.1991
(73) Patentinhaber: JUST, Hansjörg, D-79100 Freiburg (DE); Solzbach, Ulrich, Los Angeles, CA 90064 (US)
(72) Erfinder: JUST, Hansjörg, D-79100 Freiburg (DE); Solzbach, Ulrich, Los Angeles, CA 90064 (US)
(74) Vertreter: Schmitt, Hans, Dipl.-Ing.
(86) Internationale Anmeldenummer: DE8900408
(87) Internationale Veröffentlichungsnummer: WO8912478

(56) Entgegenhaltungen:
- US-A- 3 426 758
- US-A- 4 423 725
- US-A-32 799 96

## Beschreibung

Die Erfindung betrifft einen Katheter gemäß dem Oberbegriff des Hauptanspruches.

In der Medizin, speziell in der Radiologie und Kardiologie, ist die Aufdehnung (Dilatation) von Verengungen (Stenosen) entlang der Arterien eine inzwischen oft benutzte und weitgehend erfolgreiche Methode, um den Blutfluß und damit die Sauerstoff- und Energieversorgung im peripheren Gefäßbett zu erhöhen.
Dabei wird ein sogenannter Dilatationskatheter in das entsprechende stenosierte Gefäßsegment vorgeschoben und dort der am vorderen Ende befindliche Ballon aufgeweitet.
Eine häufige Komplikation bei der Aufdehnung von Gefäßveengungen ist das Auftreten von Thromben im Gefäß. Dies hat insbesondere im Bereich des Herzens für den Patienten oft gravirende Folgen, weshalb nachfolgend die Problematik insbesondere von der Koronarsituation aus beschrieben wird.

Um diese Komplikationen zu reduzieren ist es bekannt, während, vor und nach der Aufdehnung verschiedene Medikamente, u.a. Lysemittel, die Thromben wieder auflösen sollen, intrakoronar (proximal und distal des Ballons) zu verabreichen. Zum Zeitpunkt der eigentlichen Aufdehnung und der dadurch unter Umständen induzierten intrakoronaren Thrombenbildung können diese Medikamente jedoch wegen des aufgeweiteten Ballons nicht zum gewünschten Wirkort vordringen. Erst nach dem Verkleinern des Ballons bzw. nach dessen Zurückziehen aus dem aufgeweiteten Stenosebereich können die Medikamente tatsächlich an den Dilatationsbereich gelangen. Unter Umständen kann es dann aber für einen wirkungsvollen Einsatz schon zu spät sein.
Thromben, die sich z.B. im Bereich von Dissektionen gebildet haben, können leicht in die Peripherie embolisieren bzw. Ausgangspunkt für einen thrombotischen Spontanverschluß sein. Auch können Läsionen die Anfälligkeit der Gefäßinnenschicht für Gerinselbildung erhöhen.

Es ist aus der DE-OS 32 35 974 bereits ein Katheter bekannt, mit dem Medikamente in einem Stenosebereich während der Liegezeit des Katheters verabreicht werden können. Dabei sind beidseits einer Stenose aufweitbare Ballonkörper angeordnet, die den Stenosebereich nach vorne und hinten abgrenzen. In den dazwischen befindlichen Bereich wird dann durch Öffnungen in der Katheterwand ein Medikament eingebracht. Nachteilig ist hierbei, daß das Medikament nur solange einwirken kann, solange der Katheter sich im Gefäß befindet, was vergleichweise lange Liegezeiten mit entsprechenden Nachteilen ergibt. Das Einwirken des Medikaments in die Wand selbst ist hierbei nur sehr unzureichend, da die Gefäßwand in der Regel unbeschädigt ist. Man ist deshalb auf aktive Stoffwechselvorgänge und auf Diffusionsvorgänge angewiesen, die vergleichsweise viel Zeit erfordern. Dies ist besonders deshalb nachteilig, weil ein Katheter nur vergleichsweise kurze Zeit z.B. Sekunden bis höchstens wenige Minuten das Gefäß quasi verstopfen darf. Innerhalb dieser kurzen Zeit ist jedoch mit dem Katheter der DE-OS 32 35 974 eine wirksame Behandlung fraglich.

Auch die US-A- 4 423 725 zeigt eine Anordnung mit mehreren Ballonkörpern, von denen zwei beidseits einer zu behandelnden Stelle aufgeweitet werden können und dann den dazwischen liegenden Bereich abdichten, Zusätzlich ist ein dritter, zwischen den Abdichtballons angeordneter, weiterer Ballon vorgesehen, der einen medikamentösen Wirkstoff enthält, welcher in den Bereich zwischen den abdichtenden Ballons abgegeben werden kann.
Der mittlere Ballon weist dazu an seiner Oberfläche verteilt Durchtrittslöcher auf, durch die der Wirkstoff austreten kann und zum größten Teil in den Zwischenbereich zwischen den beiden äußeren Ballons gelangt. Auch hier ist ein Einwirken des Medikamentes direkt in die Gefäßwand während der Abgabe des Wirkstoffes nur unzureichend und es wäre deshalb eine längere Liegezeit des Katheters erforderlich, um ein Eindringen über Diffusionsvorgänge und dgl. zu erreichen. Außerdem ist der Aufbau dieses Ballonkatheters mit drei Einzelballonen kompliziert.

Schließlich kennt man aus der US-A- 3 279 996 einen implantierbaren Medikamententräger, der zur Abgabe von Wirkstoff über einen längeren Zeitraum hinweg ausgebildet ist. Dieser Medikamententräger weist einen kapselförmigen Körper mit einer porösen Außenwand auf, durch die Wirkstoff langsam hindurchdiffundieren kann. Eine Abgabe des Wirkstoffes innerhalb kurzer Zeit ist hier nicht vorgesehen und auch nicht möglich.

Aufgabe der vorliegenden Erfindung ist es, einen Katheter der eingangs erwähnten Art zu schaffen, der es ermöglicht, bei aufgeweitetem Ballon den dilatierten Gefäßbereich medikamentös zu behandeln. Die für eine wirksame Behandlung erforderliche Liegezeit des Katheters soll dabei wesentlich verkürzt werden. Außerdem soll der Anwendungsbereich des Katheters erweitert und sein Einsatz auch zur Behandlung in Hohlorganen möglich sein.

Zur Lösung dieser Aufgabe wird vorgeschlagen, daß der Ballon an den Enden seiner Längserstreckung benachbart zu einem mittleren Bereich an seiner zur Anlage an dem Hohlorgan bestimmten Außenseite Dichtbereiche aufweist und daß die Poren od. dgl. mit Abstand zu den Enden des Ballons in dem mittleren Bereich angeordnet sind.

Aus der DE-PS 35 16 830 ist zwar bereits ein Ballonkatheter bekannt, bei dem Öffnungen in der Ballonwand vorgesehen und im Bereich von Heizzonen angeordnet sind. Diese dienen jedoch zum Spülen des Ballons und sollen ein Ankleben am Gewebe verhindern. Außerdem soll dadurch eine vollständige Verdrängung des Blutes zwischen Ballon - und Gefäßwand erreicht werden.

Zum Infiltrieren oder "Einpressen" von medikamentösem Wirkstoff gezielt in einem vorgesehenen Behandlungsbereich ist dieser Katheter nicht vorgesehen und auch nicht geeignet. Die Öffnungen in der Ballonwand sind entsprechend der Vorgabe insbesondere zum Spülen des Zwischenbereiches zwischen Ballon und Gewebe angeordnet, was aber nur bei einer Wärmebehandlung mit gerader dichter Anlage am Gewebe aber nicht bei entsprechendem Anspressdruck beim Aufweiten der Stenose möglich ist.

Die Erfindung ermöglicht dagegen, daß bestimmte Wandbereiche eines Gefäßes oder Hohlorganes mit einem Wirkstoff angereichert werden können, wobei dieses Anreichern durch Infiltration der Wandung unter Druck schon unmittelbar während der Dilatation erfolgt. Während dieses Dilatationsvorganges öffnen sich beziehungsweise entstehen gezwungendermaßen kleine und größere Poren, Risse, Laesionen bishin zu Disektionen in der Gefäßinnenwandung, da das Lumen teilweise bei der Dilatation um ein Vielfaches aufgeweitet wird und die Gefäßinnenwandung diesem auf den Prozeß nur begrenzt standhalten kann. Unmittelbar nach der Dilatation können sich solche artifiziell geschaffenen Öffnungen der Wandung - be-dingt durch mechanisch-elastische Rückstellkräfte - teilweise wieder schließen.

Somit können hochwirksame Medikamente ihre Wirkung zum genau passenden Zeitpunkt und damit optimal entfalten, so daß Nebenwirkungen bei der Aufdehnung von Gefäßverengungen und dergleichen merklich verhindert werden können. Die Anordnung der Poren im mittleren Bereich mit Abstand zu den ballonenden schafft benachbart vor und hinter der Stenose Dichtbereiche, durch die weitgehend verhindert werden kann, daß im mittleren Ballonbereich austretender Wirkstoff in unerwünschter Weise zu den Seiten hin abströmen kann. Da-durch wird auch ein Druckabbau vermieden, der ein Einpressen des Wirkstoffes ins Gewebe in dem Maße, wie es hier vorge-sehen ist, beeinträchtigen würde.

Durch mechanisches Aufdehnen der Stenose bis zum Auftreten von Dissektionen öffnet sich das Gewebe, so daß der Wirk-stoff in kurzer Zeit eingepreßt werden kann. Es wird dabei etwa vergleichbar mit einer Injektion, in dem Gewebe auch ein Wirkstoff-Depot geschaffen, durch das eine wesentlich über die Liegezeit des Katheters hinausgehende Einwirkzeit möglich ist. Dies wiederum bringt speziell auch im Koronar-bereich wesentlichen Vorteil, daß die Occlusion des Gefäßes während der Dilatation nach sehr kurzer Zeit aufgehoben werden kann.

Unter Wirkstoffen werden im vorliegenden Falle in erster Linie Medikamente verstanden, die frische Thromben mit unterschiedlicher Wirksamkeit auflösen können (sogenannte Thrombolytika oder Lysemittel) und/oder die mittel- und langfristig einen Wiederverschluß verhindern können.
Außerdem können als Wirkstoff auch Substanzen aus der Gruppe der Fibrinkleber vorgesehen sein, die über lokale Gerinnungsprozesse Risse in der Intima bzw. Media der Arterie "verkleben" und somit mögliche Folgen zur Bildung eines falschen Lumens und Thrombenbildung durch den Kontakt der freigelegten tieferen Wandstrukturen mit dem Blut verhindern, Ferner ist der Einsatz von vielen gefäßwirk-samen Medikamenten (Nitro, Ca++ - Antagonisten usw.) denkbar. Mit Hilfe des erfindungsgemäßen Ballonkatheters ist gut auch eine medikamentöse Behandlung im Hohlorganen wie z.B. Blase, Gallenblase, Magen, Darm, Urether, Bronchien und dergleichen möglich, wobei auch hier der Wirkstoff direkt an der zu behandelnden Stelle appliziert werden kann.

Bei einer Ausführungsform, wo die Wandung des Ballons eine Vielzahl durchgehender, prorenartiger Löcher aufweist und sich der zu applizierende Wirkstoff innerhalb des Ballons befindet enthält das Dilatationsmedium zweckmäßigerweise das zu applizierende Medikament oder dergleichen oder ist selbst dieses Medikament.
Der Wirkstoff kann somit während der Dilatation durch die Wandung des Ballons hindurch direkt an die Innenwandung des Gefäßes oder des Hohlorganes gelangen.

Nach einer anderen Ausführungsform kann vorgesehen sein, daß innenseitig im Bereich der Ballonwandung eine oder mehrere Aufnahmekammern für den Wirkstoff vorgesehen sind, die nach außen führende, porenartige Löcher haben und nach innen dicht sind. Beim Dilatieren wird der in den Aufnahmekammern befindliche Wirkstoff durch die innerhalb des Ballons auftretende Druckerhöhung nach außen abgegeben und gelangt somit in erwünschter Weise in die Gefäß- bzw. Hohlorganinnenwand.

Eine andere Ausführungsform der Erfindung sieht vor, daß eine Vielzahl gegeneinander abgedichteter Aufnahmekammern vorgesehen sind und daß jeweils benachbarte Kammern abwechselnd unterschiedliche Wirkstoffkomponenten beinhalten.
Einerseits können dadurch mehrere unterschiedliche Wirkstoffe untergebracht und beim Aufweiten abgegeben werden. Auch besteht dadurch die Möglichkeit, bei Verwendung von Klebstoff, Mehr-Komponentenkleber zu verwenden, wobei in den einzelnen Aufnahmekammern die unterschiedlichen Komponenten abwechselnd untergebracht sind und beim Austreten während des Dilatierens erst zusammenkommen und aktiviert werden.

Eine andere Ausführungsform sieht vor, daß die Ballonaußenseite zwischen den Dichtbereichen eine poröse, schwammartige, Wirkstoff enthaltende, und beim Andrücken an die Gefäß- oder Hohlorganwand wenigstebs teilweise auspreßbare Schicht trägt. Der Wirkstoff wird hierbei aus der Außenschicht beim Anpressen an die Gefäß- oder Hohlorganinnenwand freigesetzt und somit insbesondere auch im Bereich des größter Gegendruckes freigesetzt.

Aus der DE-OS 23 11 807 ist zwar ein Urin-Katneter mit einem schwammartig aufweitenden Bereich bekannt. Dieser schwammartige Bereich dient aber zum Festlegen des Katneters, wenn der schwammartige Bereich mit Flüssigkeit in Berührung kommt und auch dazu, einen Abschluß zu schaffen, durch den aufsteigende Infektionen verhindert werden können. Zum Einbringen von Medikamenten in die Gefäßwand bzw. zum Demonieren von Medikamenten in den Schwamm, die dann durch Aufweiten in die Gefäßwand eingepreßt werden, ist dieser Katheter nicht geeignet.

Eine wiederum andere Ausführungsform der Erfindung sieht vor, daß der Ballon außenseitig eine abtrennbare, mit der Gefäß- oder Hohlorganinnenwand vorzugsweise verbindbare und den Wirkstoff enthaltende Ringschicht aufweist. Beim Dilatieren wird die Ringschicht gegen die Gefäß- oder Hohlorganinnenwand gepreßt und gegebenenfalls mit dieser verklebt. Beim Zusammenziehen des Ballons erfolgt eine Trennung zwischen der Ballonaußenhülle und der Ringschicht, so daß dann der Ballon zurückgezogen werden kann. Die Ringschicht verbleibt im Behandlungsbereich und kann dort ihren Wirkstoff abgeben.
Eine zweckmäßige Weiterbildung sieht vor, daß die Ringschicht u. dgl. aus resorbierbarem Material besteht. Dies hat den Vorteil, daß langfristig keine Fremdkörper z. B. im Koronarsystem verbleiben und daß das in die Arterie oder dgl. implantierte, tunnelähnliche Gebilde nach einiger Zeit abgebaut und dann nicht mehr vorhanden ist. Fremdkörper-Komplikationen werden dadurch vermieden.

Zusätzliche Ausgestaltungen der Erfindung sind in den weiteren Unteransprüchen aufgeführt. Nachstehend ist die Erfindung mit ihren wesentlichen Einzelheiten anhand der Zeichnungen noch näher erläutert. Es zeigt:
Fig. 1 eine Längsschnittdarstellung eines Gefäßabschnittes und darin befindlichem Ballonkatheter,
Fig. 2 eine schematische Außenansicht eines Ballons eines Ballonkatheters,
Fig. 3 und 4 Teilschnittdarstellungen einer Ballonaußenwand in zusammengelegter Lage bzw, aufgeweiteter Lage des Ballons,
Fig. 5 eine schematische Seitenansicht eines Ballons mit darin befindlichen Aufnahmekammern für Wirkstoff,
Fig. 6 eine abgewandelte Ausführungsform eines schematisch dargestellten Ballons mit darin befindlichen Aufnahmekammern,
Fig. 7 einen Ballonwandabschnitt mit außenseitig angebrachter, schwammartiger Schicht,
Fig. 8 eine vergrößerte Detaildarstellung von Fig. 7,
Fig. 9 eine Teillängsschnitt-Darstellung eines aufgeweiteten Ballons und außenseitig befindlicher, abtrennbarer Ringschicht und
Fig. 10 eine Darstellung eines Gefäßabschnittes mit dilatiertem Stenosebereich und darin befindlicher Ringschicht.

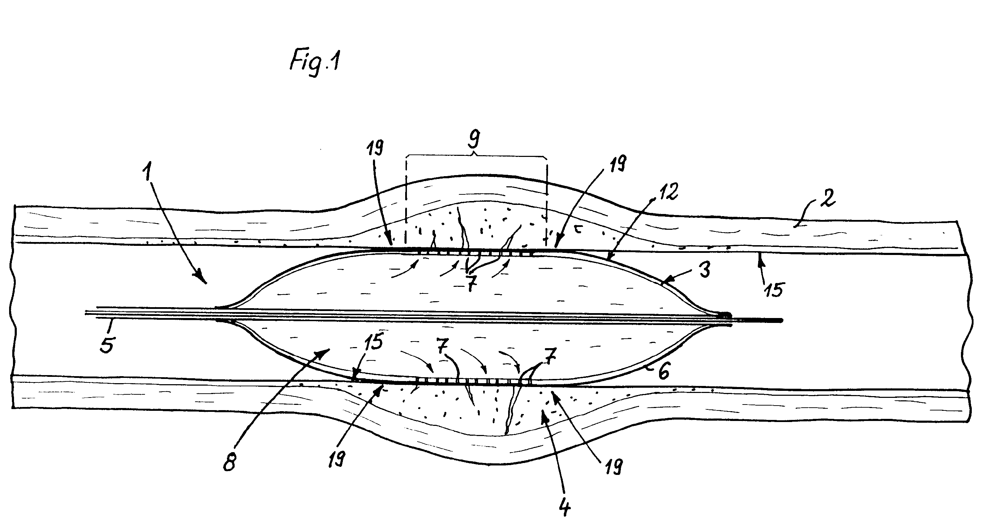

Ein in Fig. 1 in einem Gefäß 2 befindlicher Dilatationskatheter 1 weist an seinem inneren Katheterende einen Ballon 3 auf, der im Bereich einer Stenose 4 aufgeweitet wurde. Das Aufweiten des Ballons 3 erfolgt mittels eines über den Katheterschaft 5 zuführbares Dilatationsmediums (Gas oder Flüssigkeit).

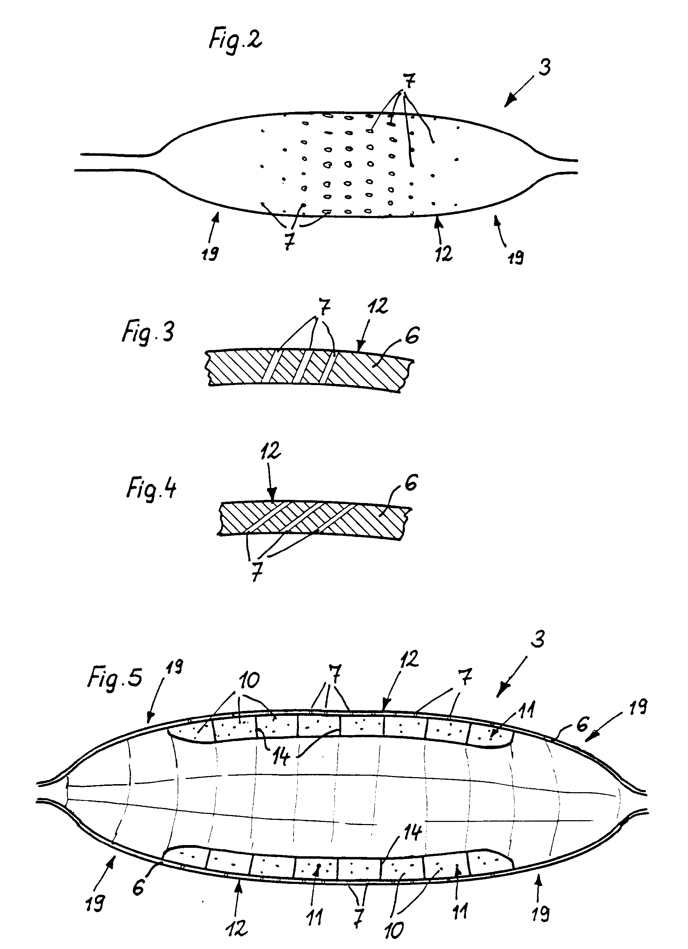

Erfindungsgemäß ist vorgesehen, daß der Ballon 3 Träger für einen z. B. in einem Stenosenbereich zu applizierenden Wirkstoff ist. Unter Wirkstoff werden im Rahmen dieser Erfindung insbesonderes Medikamente, Thrombolytika und Klebstoff verstanden.
In dem in Fig. 1 gezeigten Ausführungsbeispiel weist die Wandung 6 des Ballons 3 eine Vielzahl durchgehender, porenartiger Löcher 7 auf, so daß beim Dilatieren innerhalb des Ballons befindlicher Wirkstoff austreten und im Stenosenbereich wirksam werden kann. Bei dieser Ausführungsform ist vorgesehen, daß das Dilatationsmedium 8 das zu applizierende Medikament enthält oder aber selbst dieses Medikament ist. Die Löcher 7 sind so bemessen, daß erst beim Aufweiten des Ballons Wirkstoff enthaltendes Dilatationsmedium 8 austreten kann. In der Regel erfolgt dies somit erst aber einem bestimmten Innendruck, der sich erst beim tatsächlichen Aufweiten der Stenose einstellt. Der Wirkstoff gelangt dabei direkt an die Gefäßinnenwand im Stenosebereich, so daß dort gegebenenfalls auftretende Dissektionen unmittelbar medikamentös behandelt werden. Insbesondere kann dadurch ein "Vorfallen" von Stenoseteilen ins Innere des Gefäßes mit Bildung eines falschen Lumens und auch eine Thrombenbildung erheblich reduziert werden, da das Medikament bis zum Zurückziehen des Ballons genügend einwirken konnte, um diese Komplikationen zu verhindern. Die Wirkung des applizierten Medikamentes od.dgl. erstreckt sich durch die Infiltration der Wand auch auf tieferliegende Wand- bzw. Gewebeschichten.
Das Medikament bzw. der Wirkstoff soll ausschließlich oder vorwiegend im Bereich der Kontaktfläche des Ballons 3 mit der Stenose 4 austreten. Die porenartigen Löcher 7 sind deshalb in der in Fig. 1 und auch in Fig. 2 gezeigten Ausführungsform im mittleren Bereich 9 der Ballon-Längserstreckung und mit Abstand zu seinen Enden angeordnet. Dabei besteht die Möglichkeit, daß die Löcher 7 etwa gleichmäßig verteilt auf den ringförmigen Mittelbereich des Ballons angeordnet sind (Fig 1) oder aber die Löcher können, wie in Fig. 2 gezeigt, in ihrer Anzahl und/oder ihrem Durchtrittsquerschnitt, ausgehend vom Mittelbereich, nach außen abnehmen. Der Gesamtdurchtrittsquerschnitt der Löcher in der Ballonwand 6 nimmt somit vom mittleren Bereich der Längserstreckung des Ballons zu seinen Enden hin ab.

Benachbart zu dem mittleren Bereich 9 sind Dichtbereiche 19 gebildet, die ein seitliches Abströmen von durch die Löcher 7 zugeführtem Medikament verhindern. In Fig. 1 ist dies gut zu erkennen. Der einzige Ballon 3 hat somit drei Bereiche, nämlich zwei äußere Dichtbereiche 19 und einen mittleren Abgabebereich 9 für Medikamente od.dgl.

Die Figuren 3 und 4 zeigen eine Ballon-Wandung 6 mit porenartigen Löchern 7, die schräg zur Ballonwandoberfläche verlaufen. Fig. 3 zeigt dabei die Ballonwand in zusammengelegtem Zustand des Ballons und Fig. 4 den gleichen Wandabschnitt bei aufgeweitetem Ballon. Deutlich ist erkennbar, daß hier durch Dehnung der Ballonwandung 6 eine Querschnittsverringerung der Löcher 7 eintritt, die verhindern soll, daß mit zunehmendem Druck der Medikamentenaustritt nicht im gleichen Maße zunimmt. Man erreicht somit eine Art "Sättigung" durch die der Wirkstoffaustritt relativ unabhängig von der Innendruckzunahme im Ballon ist. Erwähnt sei hierbei auch noch, daß dieser Effekt in erster Linie beim Aufweiten des Ballons erwünscht ist, wo dessen Außenseite noch nicht in einem größeren Flächenbereich an der Stenose 4 anliegt. Durch die anschließend erfolgte Anlage der Ballonwand an der Stenoseninnenseite bzw. der Gefäßinnenseite wird ein vermehrter Austritt von Medikament allein dadurch schon verhindert.
Um aber bei Anlage des Ballons im Stenosenbereich ein erleichtertes Durchtreten der Medikamente zu ermöglichen, könnten die Löcher 7 auch so angeordnet bzw. ausgebildet sein, daß mit zunehmendem Widerstand der Ballonwand durch Anlage an der Stenose eine Stauchung und somit eine Vergrößerung des Durchtrittsquerschnittes der Löcher 7 eintritt.

Erwähnt sei noch, daß der Gesamtdurchtritts-Querschnitt der Löcher in der Ballonwand sowie die auf den Ballon einwirkenden äußeren und inneren Druckverhältnisse derart aufeinander abgestimmt sind, daß es beim Dilatieren unabhängig vom Innendruck zu einem wohldefinierten Austritt des Wirkstoffes kommt mit gegebenenfalls konstanter Rate oberhalb eines bestimmten Druckes. Da es sich bei den Löchern 7 um feine, porenartige Durchtritte handelt, ist sichergestellt, daß innerhalb des Ballons ein zum Dilatieren notwendiger Gegendruck aufgebaut werden kann.

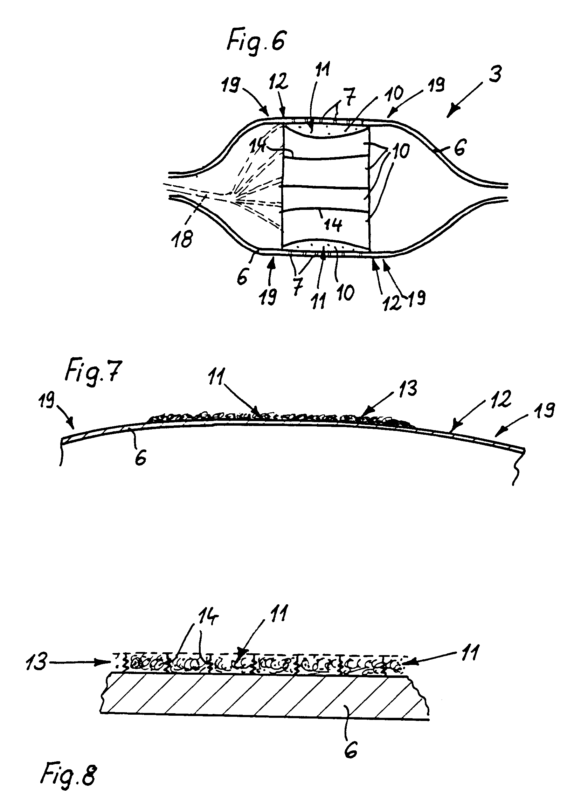

Die Figuren 5 und 6 zeigen eine abgewandelte Ausführungsform der Erfindung, wobei hier mehrere, innerhalb des Ballons 3 befindliche Aufnahmekammern 10 für den Wirkstoff vorgesehen sind. Der Wirkstoff 11 ist in den Fig. 5 bis 10 durch Punkte angedeutet.
Die Aufnahmekammern 10 sind gegeneinander und nach innen hin abgedichtet, während die Ballon-Wandung 6 im Bereich dieser Kammern 10 porenartige Löcher 7 hat. Bei dem Ausführungsbeispielen nach Fig. 5 und 6 ist ebenfalls erkennbar, daß die Aufnahmekammern 10 nur im mittleren Bereich, vergleichbar mit der Ausführungsform nach Fig. 1 und 2, vorgesehen sind. Beidseits daneben befinden sich wieder die Dichtbereiche 19.

Durch diese in Fig. 5 und 6 gezeigte Ausführungsform ist eine Trennung zwischen Dilatationsmedium und Wirkstoff vorhanden, was in einigen Anwendungsfällen wünschenswert ist. Durch den sich beim Dilatieren erhöhenden Innendruck innerhalb des Ballons 3 wird der in den Aufnahmekammern 10 befindliche Wirkstoff 11 durch die porenartigen Löcher 7 in der Wandung 6 nach außen abgegeben. Bedarfsweise könnte anstatt von mehreren, gegeneinander abgedichteten Kammern auch eine z.B. durchgehende Ringkammer vorgesehen sein, die nach innen hin zum Balloninnenraum abgedichtet ist und über Löcher 7 nach außen Verbindung hat.
Die Ausbildung mit einer oder mehreren Aufnahmekammern 10 hat auch den Vorteil, daß man eine bessere Kontrolle über die Menge der Medikamente, die austreten kann, hat. Außerdem ist dadurch auch eine geringere Menge der teilweise sehr teuren Medikamente erforderlich.
Bei einer Mehrkammerausbildung können in den einzelnen Kammern auch unterschiedliche Wirkstoffe beziehungsweise verschiedene Komponenten eines Wirkstoffes aufgenommen werden. Beispielsweise besteht dann die Möglichkeit, einen Mehrkompanentenkleber unterzubringen, dessen Komponenten erst beim Dilatieren austreten sich dann miteinander vermischen und somit aktiviert werden.

Bei Verwendung von Kleber ist ein gegenüber diesem Kleber inertes Ballonmaterial vorgesehen, um ein Festkleben des Ballons im Stenosebereich zu verhindern.

In Fig. 7 und 8 ist eine weitere mögliche Ausführungsform der Erfindung gezeigt, wobei hier die Ballonaußenseite 12 eine poröse, schwammartige, wirkstoffenthaltende Schicht 13 trägt. Diese Schicht 13 ist so aufgebaut, daß sie beim Andrücken an die Gefäßwand wenigstens teilweise auspreßbar ist und dabei den darin befindlichen Wirkstoff nach außen hin frei gibt. Auch die schwammartige Schicht 13 kann in einzelne Kammern durch Trennwände 14 unterteilt sein, um entweder verschiedene Medikamente oder verschiedene Komponenten aufnehmen zu können.

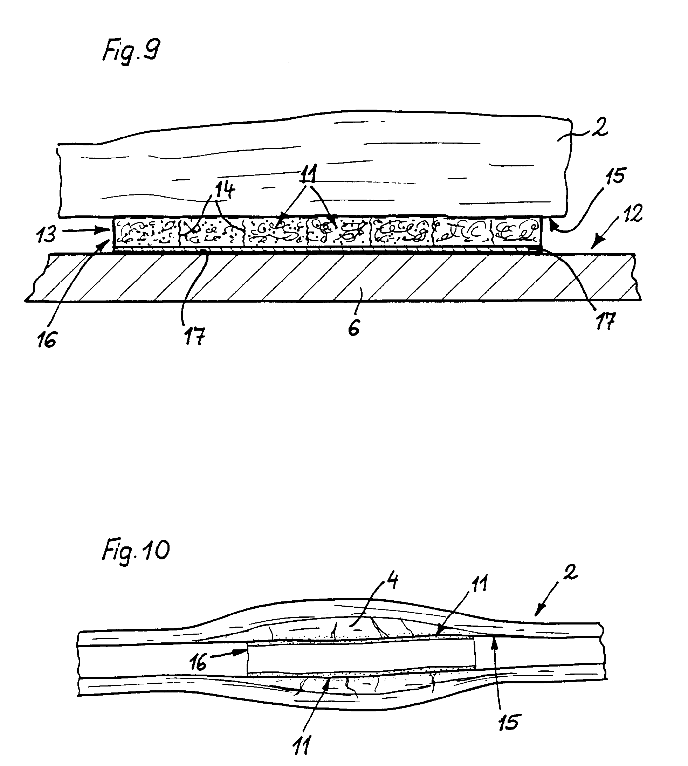

Bei der in Fig. 9 und 10 gezeigten Ausführungsform der Erfindung weist der Ballon 3 außenseitig eine abtrennbare und mit der Gefäßinnenwand 15 verbindbare Ringschicht 16 auf, welche den Wirkstoff 11 enthält.
In Fig. 9 ist der Ballon soweit aufgeweitet, daß die Ringschicht 16, welche außenseitig die poröse, schwammartige und Wirkstoff 11 enthaltende Schicht 13 aufweist, an der Gefäßinnenwand 15 anliegt. Durch weitere Druckerhöhung im Innenraum des Ballons 3 wird dann die Schicht 13 zunehmend zusammengepreßt, wodurch der darin enthaltene Wirkstoff austritt. Bei der Ausführungsform mit abtrennbarer Ringschicht 16 ist zumindest bereichsweise in der Schicht 13 Klebstoff enthalten, welcher zwischen der Gefäßinnenwand und der Ringschicht 16 eine genügend haltbare Verbindung schafft. Die Festigkeit dieser Verbindung ist so bemessen, daß sie größer ist als die zwischen der Ringschicht 16 und der Ballonwandung 6. Dadurch erfolgt ein Abtrennen der Ringschicht 16 von der Ballonaußenseite 12, sobald der Ballon zusammengezogen wird, was durch entsprechende Unterdruckbeaufschlagung im Balloninneren erfolgen kann.

Der Ballon kann nach dem Trennvorgang herausgezogen werden und die Ringschicht 16 verbleibt dann im Stenosebereich. Dadurch können auch über eine längere Zeit Wirkstoffe in die benachbarten Gefäßabschnitte abgegeben werden. Außerdem erfolgt dadurch auch eine mechanische Stabilisierung des Stenosebereiches. Eine im Stenosebereich befindliche, tunnelartige Ringschicht 16 zeigt Fig. 10, wobei hier der Dilatationskatheter bereits entfernt wurde.
Der Ballon kann außenseitig, wie in Fig. 9 angedeutet, eine der abtrennbaren Ringschicht 16 zugewandte, klebstoffinerte Trennschicht 17 aufweisen, durch die sicher verhindert wird, daß die Ringschicht 16 nach dem Klebevorgang an dem Ballon 3 hängenbleibt.

Die gesamte Ringschicht 16 mit schwammartiger Außenschicht 13 u. dgl. besteht vorzugsweise aus einem im Körper resorbierbarem Material (z.B. Zellulose), wodurch langfristig keine Fremdkörper im Koronarsystem verbleiben. Das zunächst in der Arterie implantierte, tunnelartige Trennschicht-Gebilde wäre bei dieser Ausbildung nach einiger Zeit verschwunden. Auch dadurch können Komplikationen, wie sie bei implantierten Teilen auftreten können, vermieden.

In den Figuren 1 bis 10 sind Einzelmaßnahmen zum Applizieren von Wirkstoff im Stenosebereich gezeigt. Gegebenenfalls können diese Einzelmerkmale auch kombiniert werden, wobei der Ballon beispielsweise eine poröse, schwammartige Schicht 13 mit Wirkstoff sowie eine oder mehrere, vorzugsweise neben der schwammartigen Schicht angeordnete Aufnahmekammern 10 aufweist. Auch können in Kombination mit der schwammartigen Schicht 13 perforierte Wandungsbereiche, ggf. auch zusätzlich zu Aufnahmekammern vorgesehen sein, durch die dann Wirkstoff aus dem Balloninneren nach außen treten kann.

Insgesamt erhält man durch die erfindungsgemäße Ausbildung des Dilatationskatheters 1 die Möglichkeit, während des Dilatationsvorganges direkt an die kritischen Stellen des Stenosebereiches, wo Thrombenbildung und/oder Dissektionen auftreten können, Medikamente zu verabreichen, Der Ballon kann dabei auch so lange aufgeweitet bleiben und die Stenose "in Form halten", bis die Medikamente soweit wirken, daß weitgehend gefahrlos der Ballon verkleinert und zurückgezogen werden kann. Sonst auftretende Komplikationen können dadurch weitgehend verhindert werden.
In Fig. 6 ist strichliniert noch angedeutet, daß die Aufnahmekammern 10 mit einer (ggf. auch mit mehreren) nach außen führenden Leitung 18 verbunden sind, durch die Wirkstoff in die Aufnahmekammer 10 nachgefüllt werden kann. Die Leitung 18 ist zweckmäßigerweise innerhalb des Katheterschaftes geführt.

Außer der in den Figuren gezeigten Anwendung des erfindungsgemäßen Katheters im Koronarbereich zum Aufdehnen von Verengungen, kann der Katheter auch zur Behandlung innerhalb von Hohlorganen eingesetzt werden. Beispielsweise ist eine Zytostatikagabe direkt an und in die Wand eines tumorös veränderten Hohlorganes wie Gallenwege, Darm, Harnleiter usw. möglich. Auch können Wirksubstanzen mit z.B. entzündungshemmender Wirkung auf Schleimhäute,Bronchien, Nasenschleimhäute usw. oder ätzende und adstringierende Wirkstoffe an Stellen, wo eine Lumenerweiterung sinnvoll wäre (Strikturen oder Verengungen im Bereich der Luftwege oder im Gastrointestinaltrakt) appliziert werden.
Insbesondere bei Anwendung des erfindungsgemäßen Katheters 1 im Bereich der Luftwege kann durch einen den Ballon 3 in Längsrichtung durchquerenden Bypass die eigentlich durch den Ballon blockierte Behandlungsstelle (luft-) durchlässig gehalten werden.

Alle in der Beschreibung, den Ansprüchen und der Zeichnung dargestellten Merkmale können sowohl einzeln als auch in beliebiger Kombination miteinander erfindungswesentlich sein.

## Patentansprüche

1. Katheter (1) mit einem am Katheterende befindlichen, durch ein Dilatationsmedium (8) aufweitbaren Ballon (3) zur Behandlung innerhalb von Gefäßen (2), Hohlorganen und dergleichen, insbesondere zur Behandlung von Stenosen (4) in Gefäßen (2), wobei am Ballon (3) außenseitig zur Gefäß- bzw. Hohlorganwand hin offene Poren od.dgl. für den Austritt einer Flüssigkeit beim Aufweiten des Ballons (3) vorgesehen sind, und wobei der Ballon (3) Träger für einen im Hohlorgan bzw. Gefäß (2) zu applizierenden Wirkstoff ist, **dadurch gekennzeichnet,** daß der Ballon (3) an den Enden seiner Längserstreckung benachbart zu einem mittleren Bereich (9) an seiner zur Anlage an dem Hohlorgan bestimmten Außenseite, Dichtbereiche (19) aufweist und daß die Poren (7) od. dgl. mit Abstand zu den Enden des Ballons (3) in dem mittleren Bereich (9) angordnet sind.

2. Katheter nach Anspruch 1, dadurch gekennzeichnet, daß die Poren, Löcher (7) od.dgl. etwa gleichmäßig verteilt in dem mittleren Bereich (9) angeordnet sind, vorzugsweise auf einem ringförmigen Bereich des Ballons.

3. Katheter nach Anspruch 1 oder 2, dadurch gekennzeichnet, daß die Löcher (7) in der Ballonwand (6) derart ausgebildet bzw. angeordnet sind, daß deren Querschnitt mit zunehmender Aufweitlage des Ballons abnimmt.

4. Katheter nach einem der Ansprüche 1 bis 3, dadurch gekennzeichnet, daß die Löcher (7) in der Ballonwand (6) schräg zu deren Oberfläche, ggfs. von einem geraden Durchtritt abweichend, z.B. zickzackförmig verlaufen.

5. Katheter nach einem der Ansprüche 1 bis 4, dadurch gekennzeichnet, daß der Durchtrittsquerschnitt der Löcher (7) mit zunehmendem Gegendruck von außen durch Anlage an der Gefäßwand od. dgl., insbesondere durch Stauchung zunimmt.

6. Katheter nach einem der Ansprüche 1 bis 5, dadurch gekennzeichnet, daß der Gesamtdurchtrittsquerschnitt der Löcher (7) in der Ballonwand (6) vom mittleren Bereich der Längserstreckung des Ballons zu seinen Enden hin abnimmt.

7. Katheter nach einem der Ansprüche 1 bis 6, dadurch gekennzeichnet, daß die Anzahl der Löcher (7) pro Flächeneinheit und/oder deren Durchtrittsquerschnitt vom mittleren Bereich der Ballonlängserstreckung zu seinen Enden hin abnimmt.

8. Katheter nach einem der Ansprüche 1 bis 7, dadurch gekennzeichnet, daß das Dilatationsmedium das zu applizierende Medikament od. dgl. enthält oder selbst dieses Medikament od.dgl. ist.

9. Katheter nach einem der Ansprüche 1 bis 8, dadurch gekennzeichnet, daß innenseitig im Bereich der Ballonwandung (6) mehrere Aufnahmekammern (10) für den Wirkstoff (11) vorgesehen sind ,die nach außenführende, porenartige Löcher (7) haben und nach innen dicht sind.

10. Katheter nach Anspruch 9, dadurch gekennzeichnet, daß die Aufnahmekammer (11,10) an einen nach außen insbesondere durch den Katheterschaft (5) führenden Kanal (18) angeschlossen sind.

11. Katheter nach Anspruch 9 oder 10, dadurch gekennzeichnet, daß eine oder mehrere Aufnahmekammern (10) innenseitig im perforierten Bereich der Ballonwandung vorgesehen sind.

12. Katheter nach Anspruch 9 bis 11, dadurch gekennzeichnet, daß eine Vielzahl gegeneinander abgedichteter Aufnahmekammern (10) vorgesehen sind und daß jeweils benachbarte Kammern abwechselnd ggf. unterschiedliche Wirkstoff-komponenten beinhalten.

13. Katheter nach einem der Ansprüche 1 bis 12, dadurch gekennzeichnet, daß die Ballonaußenseite (12) zwichen den Dichtbereichen (19) eine poröse, schwammartige, Wirkstoff (11) enthaltende und beim Andrücken an die Gefäß- oder Hohlorganwand od.dgl. wenigstens teilweise auspreßbare Schicht (13) trägt.

14. Katheter nach einem der Ansprüche 1 bis 13, dadurch gekennzeichnet, daß der Ballon (3) außenseitig eine abtrennbare, mit der Gefäß- oder Hohlorganinnenwand (15) vorzugsweise verbindbare und den Wirkstoff (11) enthaltende Ringschicht (16) aufweist.

15. Katheter nach Anspruch 14, dadurch gekennzeichnet, daß die Ringschicht (16) und dgl. aus resorbierbarem Material bestehen.

16. Katheter nach Anspruch 14 oder 15, dadurch gekennzeichnet, daß die abtrennbare Ringschicht (16) schwammartig und/oder mit Aufnahmekammern (10) für den bzw. die Wirkstoffe (11) ausgebildet ist und insbesondere nach innen für den Wirkstoff undurchlässig ist.

17. Katheter nach einem der Ansprüche 14 bis 16, dadurch gekennzeichnet, daß der Ballon (3) außenseitig eine der abtrennbaren Ringschicht (16) zugewandte, klebstoffinerte Trennschicht (17) aufweist.

18. Katheter nach einem der Ansprüche 1 bis 17, dadurch gekennzeichnet, daß der Ballon (3) eine poröse, schwammartige Schicht (13) mit Wirkstoff (11) sowie eine oder mehrere, vorzugsweise neben der schwammartigen Schicht (13) angeordnete Aufnahmekammern (10) aufweist.

19. Katheter nach einem der Ansprüche 1 bis 18, dadurch gekennzeichnet, daß der Ballon (3) eine poröse, schwammartige Schicht (13) mit Wirkstoff (11) sowie wenigstens einen perforierten Wandungsbereich und/oder Aufnahmekammern (10) aufweist.

## Claims

1. A catheter (1) having at the end thereof a balloon (3) expandable by a dilatation medium (8) for treatment within vessels (2), hollow organs and the like, particularly for the treatment of stenoses (4) in vessels (2), the balloon (3) being provided externally with pores or the like which are open towards the wall of the vessel or of the hollow organ and serve for a liquid to emerge when the balloon (3) expands, and the balloon (3) being a carrier for an active substance to be administered in the hollow organ or vessel (2), **characterized in that** at the ends of the longitudinal expanse of the balloon (3), adjacent to a middle zone (9), the balloon (3) has sealing areas (19) on its surface for contacting the hollow organ and that the pores (7) or the like are arranged in the middle zone (9) so as to be in spaced relationship to the ends of the balloon (3).

2. A catheter as claimed in claim 1, characterized in that the pores, apertures (7) or the like are arranged in approximately even distribution in the middle zone (9), preferably on an annular area of the balloon.

3. A catheter as claimed in claim 1 or claim 2, characterized in that the apertures (7) in the wall (6) of the balloon are configured or arranged such that their cross section decreases as the expanded condition of the balloon increases.

4. A catheter as claimed in any one of claims 1 to 3, characterized in that the apertures (7) in the wall (6) of the balloon show a development which is oblique to the wall surface and may deviate from a straight passage, e.g. a zig-zag one.

5. A catheter as claimed in any one of claims 1 to 4, characterized in that the section of passage of the apertures (7) increases with increasing counterpressure from outside by contact with the vessel wall or the like, particularly by compression.

6. A catheter as claimed in any one of claims 1 to 5, characterized in that the overall section of passage of the apertures (7) in the wall (6) of the balloon decreases from the middle zone of the longitudinal expanse of the balloon to the ends of the balloon.

7. A catheter as claimed in any one of claims 1 to 6, characterized in that the number of apertures (7) per unit of area and/or the section of passage thereof decreases from the middle zone of the longitudinal expanse of the balloon to the ends of the balloon.

8. A catheter as claimed in any one of claims 1 to 7, characterized in that the dilatation medium contains or is itself the medicament or the like to be administered.

9. A catheter as claimed in any one of claims 1 to 8, characterized in that provided interiorly in the area of the balloon wall (6) are a plurality of chambers (10) receiving the active substance (11), which chambers have pore-like apertures (7) leading outwards and are inwardly tight.

10. A catheter as claimed in claim 9, characterized in that the receiving chambers (11, 10) are connected to a duct (18) leading outwards particularly through the catheter shank (5).

11. A catheter as claimed in claim 9 or claim 10, characterized in that one or more receiving chambers (10) are provided interiorly in the perforated area of the balloon wall.

12. A catheter as claimed in any one of claims 9 to 11 , characterized in that a plurality of receiving chambers (10) sealed relative to one another are provided and that adjacent chambers may alternately contain different constituents of active substance.

13. A catheter as claimed in any one of claims 1 to 12, characterized in that the outer surface (12) of the balloon carries between the sealing areas (19) a porous, spongy layer (13) containing an active substance (11) which is at least partly squeezed out when the layer presses against the wall of the vessel or hollow organ or the like.

14. A catheter as claimed in any one of claims 1 to 13, characterized in that the balloon (3) has on its outer surface a detachable annular layer (16) which is preferably connectable to the inside wall (15) of the vessel or hollow organ and contains the active substance (11).

15. A catheter as claimed in claim 14, characterized in that the annular layer (16) and the like consist of resorbable material.

16. A catheter as claimed in claim 14 or claim 15, characterized in that the detachable annular layer (16) is devised to be spongy and/or to have receiving chambers (10) for the active substance(s) (11) and in particular is inwardly impermeable for the active substance.

17. A catheter as claimed in any one of claims 14 to 16, characterized in that the balloon (3) has on its outer surface a parting layer (17) which faces the detachable annular layer (16) and is inert with respect to adhesive.

18. A catheter as claimed in any one of claims 1 to 17, characterized in that the balloon (3) has a porous, spongy layer (13) with active substance (11) as well as one or more receiving chambers (10) preferably arranged beside the spongy layer (13).

19. A catheter as claimed in any one of claims 1 to 18, characterized in that the balloon (3) has a porous, spongy layer (13) with active substance (11) as well as at least one perforated wall area and/or receiving chambers (10).

## Revendications

1. Cathéter (1) comportant un ballonnet (3) situé à l'extrémité du cathéter et gonflable par un fluide de dilatation (8) pour des traitements à l'intérieur de vaisseaux (2), organes creux et analogues, en particulier pour le traitement de sténoses (4) dans des vaisseaux (2), avec prévision sur le côté extérieur du ballonnet (3) de pores ou analogues ouverts vers la paroi du vaisseau ou de l'organe creux pour la sortie d'un liquide lors du gonflage du ballonnet (3), et dont le ballonnet (3) constitue un véhicule pour une substance active à appliquer dans l'organe creux ou le vaisseau (2), caractérisé en ce que le ballonnet (3) présente des zones d'étanchéité (19) aux extrémités de son étendue longitudinale, à proximité d'une zone centrale (9), sur son côté extérieur destiné à être appliqué contre l'organe creux, et que les pores (7) ou analogues sont disposés dans la zone centrale (9), à distance des extrémités du ballonnet (3).

2. Cathéter selon la revendication 1, caractérisé en ce que les pores, trous (7) ou analogues sont disposés avec une répartition à peu près uniforme dans la zone centrale (9), de préférence sur une partie annulaire du ballonnet.

3. Cathéter selon la revendication 1 ou 2, caractérisé en ce que les trous (7) sont agencés ou disposés dans la paroi (6) du ballonnet de manière que leur section diminue à mesure que le gonflement du ballonnet augmente.

4. Cathéter selon une des revendications 1 à 3, caractérisé en ce que les trous (7) de la paroi (6) du ballonnet s'étendent obliquement par rapport à la surface de cette paroi, en s'écartant éventuellement d'un passage rectiligne, par exemple en ayant une allure en zigzag.

5. Cathéter selon une des revendications 1 à 4, caractérisé en ce que la section de passage des trous (7) augmente avec l'accroissement de la contre-pression de l'extérieur, par suite de l'application contre la paroi du vaisseau ou analogue, notamment par refoulement.

6. Cathéter selon une des revendications 1 à 5, caractérisé en ce que la section de passage totale des trous (7) dans la paroi (6) du ballonnet diminue à partir de la zone centrale de l'étendue longitudinale du ballonnet vers ses extrémités.

7. Cathéter selon une des revendications 1 à 6, caractérisé en ce que le nombre des trous (7) par unité de surface et/ou leur section de passage diminue depuis la zone centrale de l'étendue longitudinale du ballonnet vers ses extrémités.

8. Cathéter selon une des revendications 1 à 7, caractérisé en ce que le fluide de dilatation contient le médicament ou analogue à appliquer ou constitue lui-même ce médicament ou analogue.

9. Cathéter selon une des revendications 1 à 8, caractérisé en ce que plusieurs alvéoles réceptrices (10) pour la substance active (11) sont prévues à l'intérieur du ballonnet, dans la région de sa paroi (6), alvéoles qui présentent des trous (7) semblables à des pores menant vers l'extérieur et qui sont fermées étanches vers l'intérieur.

10. Cathéter selon la revendication 9, caractérisé en ce que les alvéoles réceptrices (11, 10) sont raccordées à un canal (18) menant à l'extérieur, notamment à travers la tige (5) du cathéter.

11. Cathéter selon la revendication 9 ou 10, caractérisé en ce qu'une ou plusieurs alvéoles réceptrices (10) sont prévues à l'intérieur du ballonnet dans la région perforée de la paroi de celui-ci.

12. Cathéter selon les revendications 9 à 11, caractérisé en ce qu'un grand nombre d'alvéoles réceptrices (10) mutuellement étanchées sont prévues les unes à côté des autres et que des alvéoles voisines contiennent alternativement des composants éventuellement différents de substance active.

13. Cathéter selon une des revendications 1 à 12, caractérisé en ce que le côté extérieur (12) du ballonnet porte, entre les zones d'étanchéité (19), une couche (13) poreuse, de type éponge, contenant une substance active (11) et dont le contenu peut être exprimé au moins partiellement lorsqu'elle est pressée contre la paroi du vaisseau, de l'organe creux ou analogue.

14. Cathéter selon une des revendications 1 à 13, caractérisé en ce que le ballonnet (3) présente sur le côté extérieur une couche annulaire (16) détachable, pouvant être reliée de préférence à la paroi interne du vaisseau ou de l'organe creux (15) et contenant la substance active (11).

15. Cathéter selon la revendication 14, caractérisé en ce que la couche annulaire (16) et des éléments semblables sont faits d'un matériau résorbable.

16. Cathéter selon la revendication 14 ou 15, caractérisé en ce que la couche annulaire (16) détachable est réalisée à la façon d'une éponge et/ou avec des alvéoles réceptrices (10) pour la ou les substances actives (11) et est notamment imperméable à la substance active vers l'intérieur.

17. Cathéter selon une des revendications 14 à 16, caractérisé en ce que le ballonnet (3) présente sur le côté extérieur une couche de séparation (17) dirigée vers la couche annulaire (16) détachable et qui est inerte vis-à-vis de la matière adhésive.

18. Cathéter selon une des revendications 1 à 17, caractérisé en ce que le ballonnet (3) présente une couche poreuse (13) de type éponge contenant une substance active (11), ainsi qu'une ou plusieurs alvéoles réceptrices (10) disposées de préférence à côté de la couche (13) de type éponge.

19. Cathéter selon une des revendications 1 à 18, caractérisé en ce que le ballonnet (3) présente une couche poreuse (13) de type éponge contenant une substance active (11), ainsi qu'au moins une région de paroi perforée et/ou des alvéoles réceptrices (10).
